(19)

Europäisches
Patentamt
European
Patent Office
Office européen
des brevets

(11)　EP 3 225 263 A1

(12)　**EUROPEAN PATENT APPLICATION**
published in accordance with Art. 153(4) EPC

(43) Date of publication:
**04.10.2017　Bulletin 2017/40**

(51) Int Cl.:
**A61M 5/142** (2006.01)

(21) Application number: **15862852.9**

(86) International application number:
**PCT/CN2015/094538**

(22) Date of filing: **13.11.2015**

(87) International publication number:
**WO 2016/082685 (02.06.2016 Gazette 2016/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.11.2014　CN 201410709653**

(71) Applicant: **Zensun (Shanghai) Science and
Technology Limited
Shanghai 201203 (CN)**

(72) Inventor: **ZHOU, Mingdong
Bexley, New South Wales 2207 (AU)**

(74) Representative: **Schorr, Peter Karl
Schorr IP
Patentanwaltskanzlei
Am Sonnenweg 20
70619 Stuttgart (DE)**

(54)　**INJECTION PUMP CONTROL METHOD, CONTROL UNIT, AND INJECTION PUMP**

(57)　A method for controlling an insulin injection pump, a related control unit (200), and an injection pump (300). The control unit (200) comprises: an expected plasma insulin concentration value acquiring module (202), a current subcutaneous and interstitial insulin concentration value acquiring module (201), a supposed injection value acquiring module (203), an injection instruction sending module (204), and a counting module (205). The injection pump comprises a pump body and the control unit (200), and the control unit (200) is disposed in the pump body.

Figure 5

## Description

### Technical Field

The present invention relates to a method of controlling an injection pump, and further relates to a related control unit and an injection pump.

### Background

Diabetes is a chronic disease that heavily endangers human health in the modem society. In practice, it is most effective to use insulin to control blood glucose and prevent and control various serious complications. Since insulin cannot be taken orally, it is conventional to use an injector to inject insulin. Currently, there are also increasingly more examples of injection using injection pumps.

An injection pump is used to accurately inject insulin at a fixed frequency in a fixed quantity so as to achieve the purposes of better controlling the blood glucose concentration in diabetic patients, reducing the fluctuation of the blood glucose, and reducing the occurrence of hypoglycemia, thereby improving the patients' quality of life. The presence of an injection pump was once considered as good news for diabetic patients. However, popularity of injection pumps is at a low level at present. Apart from factors such as high costs, the trouble caused to the medical workers and patients by tedious process of setting an injection dosage of a pump also restricts the application and promotion of the injection pumps.

Currently, the dosage of an injection pump is set according to a scheme of "total dosage - basic dosage - additional dosage", i.e., the total amount of insulin is calculated according to the weight and conditions of a patient, or the past usage and the therapeutic effect of the insulin; the total dosage is multiplied by a distribution coefficient to respectively obtain a basic dosage and a before meal (during meal) additional dosage. However, the method cannot enable a patient's blood glucose concentration to rapidly reach an expected range. Usually, four times of monitoring of the blood glucose are needed every day, and the amount of insulin is constantly adjusted according to the blood glucose concentration. The operation of initially setting the dosage of an injection pump is usually accomplished by medical staff In order to find out a comparatively satisfactory amount of insulin, the diabetic patients are often required to stay in hospital for a week or so; if the patients choose not to stay in hospital, then it will take longer time to accomplish the setting of the dosage of the injection pump. Additionally, since the kinds and times of meal for a patient might change every day, it then requires the user of the pump to acquire the calculation method of the before meal/during meal additional amount. In this regard, it sets out relatively higher requirements for users' learning ability. However, many patients abandon pump treatment because of disability to accommodate and acquire the treatment. On the other hand, there are various insulin preparations that can be chosen by clinical doctors during conventional treatment, including rapid-acting, short-acting, intermediate-acting and long-acting insulin. Each preparation has its pharmacokinetic/pharmacodynamic characteristics and indications, but the injection pumps only employ rapid-acting or short-acting insulin. Such scheme of setting differs significantly from daily therapeutic scheme, and the medical staff need to be trained to acquire the setting method. Nevertheless, most medical staff do not understand the scheme of setting such kind of injection pumps, which also becomes a restrictive factor of the application of injection pumps.

### Summary

With respect to the abovementioned problems, the present invention proposes a solution. According to the present invention, the use of rapid-acting or short-acting preparation in combination with an injection pump to simulate the pharmacokinetic/pharmacodynamic characteristics of long-acting or intermediate-acting preparations extremely facilitates the medical staff and the patients to use the injection pumps, thereby promoting popularization of the injection pumps and benefiting the majority of patients. Apart from the method of controlling an injection pump, the present invention further provides a control unit for the injection pump and an injection pump comprising the control unit correspondingly.

According to an aspect of the present invention, a method of controlling an injection pump for injecting preparations is provided. The method comprises: (1) acquiring a current first location preparation concentration value of an individual to be injected with a preparation at a current moment; (2) acquiring an expected concentration value in plasma at a predetermined next moment based on a first injection scheme; (3) acquiring a supposed injection value at a current moment based on a second injection scheme, an expected concentration value in plasma at the next moment, and a current first location preparation concentration value; (4) injecting a preparation into an individual using the pump according to the supposed injection value; and (5) repeating steps (1) to (4) until the time experienced from a first injection reaches a lasting time length of the first injection scheme.

Preferably, when there is no preparation for injection, a first location preparation concentration value is 0.

Preferably, in step (1), a current second location preparation concentration value at a current moment is further obtained, and in step (3) a supposed injection value at a current moment is also obtained based on the current second location preparation concentration value.

Preferably, when there is no preparation for injection, a second location preparation concentration value is 0.

Preferably, the first injection scheme is a long-acting or intermediate-acting injection scheme.

Preferably, the second injection scheme is a short-acting or rapid-acting injection scheme.

Preferably, the first location is a subcutaneous location.

Preferably, the second location is interstice.

Preferably, the preparation is insulin.

Preferably, the displacement distance of a pump is monitored in real time when a pump is used to inject a preparation into an individual. In this way, an injection amount of a pump can be controlled accurately.

According to another aspect of the present invention, a control unit for controlling an injection pump to inject a preparation is provided, which comprises: a current concentration value acquiring module, which is configured to obtain a current first location preparation concentration value of an individual to be injected with a preparation at a current moment; an expected concentration value acquiring module which is configured to obtain an expected concentration value at a predetermined next moment based on a first injection scheme; a supposed injection value acquiring module which is configured to obtain a supposed injection value at a current moment based on a second injection scheme, an expected concentration value at the next moment, and the current first location preparation concentration value; an injection instruction sending module which is configured to send instructions to inject a preparation into an individual using the pump according to the supposed injection value; and a time counting module which is configured to send out a signal to stop injection when the time experienced from the first injection reaches the lasting time length of the first injection scheme.

Preferably, the expected concentration value acquiring module further acquires a current second location preparation concentration value at the current moment, and the supposed injection value acquiring module further acquires a supposed injection value at the current moment based on a current second location preparation concentration value.

Preferably, a time counting module is achieved through a counter, wherein when the times of predetermined injection are achieved, the time counting module sends out a signal to stop injection.

Preferably, a control unit further comprises a displacement transducer for monitoring a displacement distance of a pump in real time.

According to a further aspect of the present invention, an injection pump for injecting a preparation is provided, the injection pump comprising a pump body and a control unit as stated in the previous paragraph, wherein the control unit is arranged in the pump body.

According to an aspect of the present invention, a method of controlling an insulin injection pump is provided. The method comprises: (1) obtaining a current subcutaneous insulin value and a current interstitial insulin value of an individual to be injected with insulin at a current moment; (2) obtaining an expected plasma insulin concentration value at a predetermined next moment based on a first insulin injection scheme; (3) calculating a supposed injection value at a current moment based on a second insulin injection scheme, a current subcutaneous insulin value, a current interstitial insulin value, and an expected plasma insulin concentration value at a next moment; (4) injecting insulin into an individual using the pump according to the supposed injection value; and (5) repeating steps (1) to (4) until the time experienced from the first injection reaches a lasting time length of the first insulin injection scheme.

Preferably, when there is no insulin injection, the subcutaneous insulin value and the interstitial insulin value are 0.

Preferably, a current subcutaneous insulin value at a current moment is obtained first, and then a current interstitial insulin value is obtained based on the current subcutaneous insulin value.

Preferably, an insulin value refers to a monomeric/dimeric insulin value.

Preferably, a first injection scheme is a long-acting or intermediate-acting injection scheme.

Preferably, a second injection scheme is a short-acting or rapid-acting injection scheme.

Preferably, when an individual is injected with insulin using the pump, the displacement distance of the pump is monitored in real time. In this way, the injection amount of a pump can be accurately controlled.

According to another aspect of the present invention, a control unit for controlling an insulin injection pump is provided, the control unit comprising: a current concentration value acquiring module, which is configured to obtain with a current interstitial insulin value and a current subcutaneous insulin value at a current moment; an expected concentration value acquiring module, which is configured to obtain an expected plasma insulin concentration value at a predetermined next moment based on a first insulin injection scheme; a supposed injection value acquiring module, which is configured to obtain a supposed injection value at a current moment based on a second insulin injection scheme, an expected plasma insulin concentration value at the next moment, a current subcutaneous insulin value and a current interstitial insulin value; an injection instruction sending module, which is configured to send out an instruction to inject insulin into the individual using the pump according to the supposed injection value; and a time counting module, which is configured to send out a signal to stop injection when the time experienced from the first injection reaches the lasting time length of the first injection scheme.

Preferably, the time counting module is realized through a counter, wherein when a predetermined injection times are achieved, the time counting module sends out a signal to stop injection.

Preferably, a control unit further comprises a displacement transducer for monitoring a displacement distance of a pump in real time.

According to a further aspect of the present invention, an injection pump for injecting insulin is provided, the injection pump comprising a pump body and the control unit as stated in the previous paragraph, wherein the control unit is arranged in the pump body.

According to an aspect of the present invention, a method of controlling an insulin injection pump is provided. The method comprises: (1) obtaining a first expected plasma insulin concentration value of an individual to be injected with insulin at a predetermined first moment based on a first insulin injection scheme; (2) calculating an initial supposed injection value at an initial moment based on a second insulin injection scheme and the first expected plasma insulin concentration value; (3) injecting insulin into an individual using the pump according to the initial supposed injection value; (4) obtaining a first moment interstitial insulin value and a first moment subcutaneous monomeric/dimeric insulin value at the first moment based on a second insulin injection scheme; (5) obtaining a second expected plasma insulin concentration value at a predetermined second moment based on the first insulin injection scheme; (6) calculating a first supposed injection value at a first moment based on the second insulin injection scheme, the second expected plasma insulin concentration value and the first moment subcutaneous monomeric/dimeric insulin value and the first moment interstitial insulin value; (7) injecting insulin into an individual using the pump according to the first supposed injection value; and (8) obtaining a supposed injection value at each moment by iteration based on steps (4) to (7) and injecting insulin into the individual using the pump according to the supposed injection value until the time experienced from a first injection reaches a lasting time length of the first insulin injection scheme.

Preferably, an interval between a first moment and an initial moment is equal to an interval between a second moment and the first moment.

Preferably, the time interval between every two injections is fixed.

Preferably, the first injection scheme is a long-acting or intermediate-acting injection scheme.

Preferably, the second injection scheme is a short-acting or rapid-acting injection scheme.

According to another major aspect of the present invention, a control unit for controlling an insulin injection pump is provided. The control unit comprises: an expected plasma insulin concentration value acquiring module, which is configured to obtain a first expected plasma insulin concentration value at a predetermined first moment and a second expected plasma insulin concentration value at a predetermined second moment of an individual to be injected with insulin based on the first insulin injection scheme, until an N-th expected plasma insulin concentration value at a predetermined N-th moment is reached; wherein N refers to the number of times of predetermined injections; a current subcutaneous and interstitial insulin concentration value acquiring module, which is configured to obtain with a current subcutaneous insulin value and a current interstitial insulin value at a current moment; a supposed injection value acquiring module, which is configured to obtain a supposed injection value at a current moment based on a second insulin injection scheme, an expected plasma insulin concentration value at a next moment, the current subcutaneous insulin value and the current interstitial insulin value; an injection instruction sending module, which is configured to send out an instruction to inject insulin into an individual using the pump according to the supposed injection value; and a counting module, which is configured to send out a signal to stop injection when the time experienced from the first injection reaches the lasting time length of the first injection scheme.

Preferably, a time counting module is achieved through a counter, wherein the time counting module sends out a signal to stop injection when predetermined injection times are accomplished.

Preferably, a control unit further comprises a displacement transducer for monitoring a displacement distance of a pump in real time.

According to a further aspect of the present invention, an injection pump for injecting inulin is provided, the injection pump comprising a pump body and the control unit as stated in the previous paragraph, wherein the control unit is arranged in the pump body.

It should be understood that the technical solution of the present invention is not only limited to the abovementioned as listed. The features of all the abovementioned major technical solutions and all the preferable schemes can be combined freely as long as the combined features are not conflicting with each other therebetween.

**Brief Description of the Drawings**

Figure 1 is a flow chart of a method of controlling an injection pump according to an embodiment mode of the present invention;

Figure 2 is a curve diagram of plasma concentration variation of a patient weighed 70kg when subcutaneously injected with 10IU NPH;

Figure 3 is a curve diagram of plasma concentration variations of pump injection with rapid-acting insulin and one shot of injection with 10IU intermediate-acting insulin, wherein local details are further displayed in the form of an enlarged view;

Figure 4 is a functional diagram of a control unit for an injection pump according to an embodiment mode of the present invention;

Figure 5 is a schematic diagram of an injection pump according to an embodiment mode of the present invention;

Figure 6 shows injection amounts and theoretically expected value of an injection pump every 15 minutes according to an example of the present invention;

Figure 7 is a PK diagram comparing NPH single subcutaneous injection and successively subcutaneous injection using an injection pump with a blank control group according to an example of the present invention.

The specific embodiments of the present invention are set forth hereinafter in combination with the drawings. It should be understood that these embodiment modes are illustrative rather than restrictive.

**Description of the Embodiments**

The present invention is described using some examples in the description, wherein some preferable embodiment modes are included. It should be understood that the scope of protection of the present invention further comprises other examples.

For example, a system and method may comprise a data signal transmitted via a network (e.g., a local area network, a wide area network, an Internet, and their combination, etc.), an optical fiber medium, a carrier, a wireless network or the like for communicating with one or more data processing units. The data signal can carry any or all data disclosed in the text. In addition, the method and system described herein can also be realized on different types of processing units by executing program codes comprising instructions on processing subsystems of these units. Software program instructions may comprise source codes, target codes, machine codes or other stored data, which can be operable to enable the processing system to execute the method and operation described herein. Other embodiment modes are also feasible, e.g., firmware or even suitable hardware that can execute the method and system as described in this text is arranged.

Data (e.g., relevance, mapping, data input, data output, intermediate data results, final data results and the like) of the system and method can be stored and executed on one or more different types of non-volatile computer readable storage media, wherein the media can be in a certain location or distributed in different locations. The media may comprise data storage units that are implemented by computers, e.g., different types of storage units and programming structures (e.g., RAM, ROM, flash memory, flat files, database, programming data structures, programming variables, IF-THEN (or similar) declaration structures, etc.). It should be understood that data structure describes format for organizing and storing data, database, programs, memory or other computer readable media that can be used by computer programs.

The system and method can be provided on many different types of computer readable media, including computer storage mechanical structures (e.g., CD-ROM, disks, RAM, flash memory, computer hard disk and the like) which comprise instructions (e.g., software) to be executed by processors so as to complete the operation of the method described herein and realize the system.

The computer components, software modules, functions, data storage and data structures described herein can be directly or indirectly connected to one another to allow data flow required in the work. It should be further understood that the module or processor not only comprises such code units that execute software operations, but also can be realized as a subprogram unit, as a software functional unit of codes, as an object (in an object-oriented normal form), as an applet, in a computer script language, or as another type of computer codes. The software components and/or functions can be located on a single computer or distributed on a plurality of computers according to different conditions.

It should be understood that the components used in the present description and mentioned in the claims can be in a single form or in a plural form. Similarly, "in ..." as used in the present description and mentioned in the claims comprises two meanings, i.e., "in ..." and "on ..." unless specifically indicated in the context. Finally, "and" and "or" as used in the present description and mentioned in the claims comprise two meanings, i.e., connective and separated unless specifically indicated in the context. They can be used interchangeably. The term "exclusive or" can be used to indicate the condition of using the meaning of separation only.

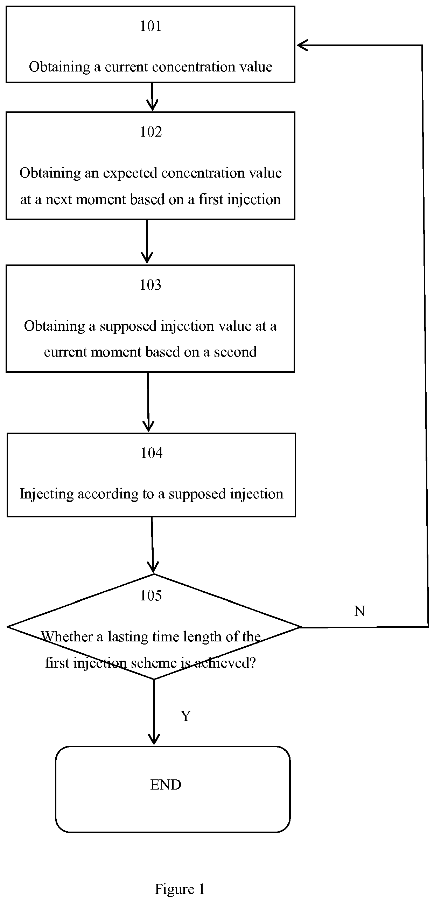

Figure 1 presents a flow chart of a method of controlling an injection pump according to an embodiment mode of the present invention. As illustrated in the figure, the method includes five steps. First of all, a preparation concentration value at a current first location and a preparation concentration value at a current second location of an individual to be injected with a preparation at a current moment are obtained in step 101. Then, in step 102, an expected concentration value at a predetermined next moment is obtained based on a first injection scheme. Next, in step 103, a supposed injection value at a current moment is obtained based on a second injection scheme, an expected concentration value at a next moment, a preparation concentration value at a current first location and a preparation concentration value at a current second location. Further, in step 104, a preparation is injected into an individual using the pump according to the supposed injection value. Next, in step 105, it is determined as to whether or not the time experienced from the first injection reaches a lasting time length of the first injection scheme. If yes, it comes to an end; if no, it comes back to step 101 to execute the process again successively, i.e., steps 101 to 104 are repeated until the time experienced from the first injection reaches the lasting time length of the first injection scheme. Herein, the so-called lasting time length of the first injection scheme refers to the lasting time length in which the preparation takes effect when injection is executed according to the first injection scheme.

It should be understood that in other embodiments of the present invention, it is possible to only obtain a preparation concentration value at a first location or at a second location, rather than obtain both preparation concentration values at a first location and a second location.

In a preferred embodiment mode, the first location can be a subcutaneous location, and the second location can be interstitice. The first injection scheme is a long-acting injection scheme, and the second injection scheme is a rapid-acting injection scheme, with insulin as the preparation.

In particular, with respect to insulin injection, the present invention can be transformed into rapid-acting injection scheme that is suitable for injection using an injection pump based on pharmacokinetic principles according to conventional prescriptions of insulin, in which clinical prescription schemes and clinical experience of insulin acquired by doctors are fully utilized to increase accuracy and reliability of the prescriptions. The method of using the pump is simplified, the time of setting and adjusting dosage is reduced, and the time spent on using insulin for doctors and patients is saved. Therefore, insulin is taken as an example hereinafter to describe a particularly specific embodiment mode of the present invention.

At present, there are various types of insulin that are used clinically. The usual mode of administration is subcutaneous injection, but the effect features during action differ significantly. The short-acting insulin (RI) takes effect from 15 to 60 minutes after injection, the rapid-acting insulin from 10 to 15 minutes, the intermediate-acting (NPH, lent) insulin from 2.5 to 3 hours, and the long-acting (ultralente, glargine) insulin from 2 to 3 hours, wherein the effect can last for 20 to 30 hours. The major factor that causes differing time of action lies in the difference in the process of subcutaneous absorption of insulin, which process generally comprises the following stages: in subcutaneous tissues, crystalline insulin is dissolved into hexamer which can be depolymerized into dimer/monomer. The latter can be absorbed into blood after entering into interstice subcutaneously. Each stage of absorption follows a first-order kinetic mode.

In this example, it is set that a conventional therapeutic solution is 10IU NPH/day. Moreover, the patient weighs 70kg, and rapid-acting insulin, e.g., NovoRapid from Novo Nordisk, is used in an insulin pump.

First of all, the pharmacokinetic features of intermediate-acting insulin NPH (e.g., Novolin from Novo Nordisk) are calculated.

The process of subcutaneous absorption of NPH is described using differential equations:

$$u_{total,NPH}(t) = u_{c,NPH}(t) + u_{h,NPH}(t) + u_{m,NPH}(t) \qquad (1)$$

$$u_{c,NPH}(t) = \alpha_{NPH} u_{total,NPH}(t) \qquad (2)$$

$$\dot{C}_{NPH}(t) = - k_{crys,NPH} C_{NPH}(t) + u_{c,NPH}(t) \qquad (3)$$

$$\dot{x}_h(t) = -(k_1 + k_d)x_h(t) + k_{crys,NPH} C_{NPH}(t) + u_{h,NPH}(t) \qquad (4)$$

$$\dot{x}_{dm}(t) = -(k_2+k_d)x_{dm}(t)+k_1x_h(t) +u_{m,NPH}(t) \qquad (5)$$

$$\dot{x}_i(t) = -(k_3+k_{d,i})x_i(t)+k_2x_{dm}(t) \qquad (6)$$

$$\dot{I}(t) = -nI(t)+k3\left\{\frac{x_i(t)}{V_im_b}\right\} \qquad (7)$$

Therein,

$u_{total,NPH}(t)$: a total rate (mU/min) of injecting intermediate-acting insulin NPH;

$u_{c,NPH}(t)$: a rate (mU/min) of injecting crystalline NPH insulin;

$u_{h,NPH}(t)$:: a rate (mU/min) of injecting hexameric NPH insulin;

$u_{m,NPH}(t)$: a rate (mU/min) of injecting monomeric/dimeric NPH insulin;

$\alpha_{NPH}$: a ratio of crystalline insulin to total amount of NPH in NPH;

$C_{NPH}(t)$: an amount (mU) of subcutaneously crystalline NPH;

$k_{crys,NPH}$: a rate (min$^{-1}$) of dissolving NPH crystals;

$x_h$: an amount (mU) of hexameric insulin in subcutaneous tissues;

$k_1$: a rate (min$^{-1}$) of depolymerizing hexamers;

$k_2$: a rate (min$^{-1}$) of transporting dimeric/monomeric insulin from subcutaneously to interstitially;

$k_d$: a rate (min$^{-1}$) of consumption of hexamers, dimers and monomers owing to diffusion;

$x_{dm}$: an amount (mU) of hexameric insulin;

$k_3$: a rate (min$^{-1}$) of transporting insulin from interstitially to plasma;

$k_{d,i}$: a rate of consumption or loss interstitially, e.g., 0.0029 (min$^{-1}$);

$I(t)$: a plasma insulin concentration (mU/L);

$n$: a rate of removing plasma insulin, e.g., 0.16 (min$^{-1}$);

$V_i$: a volume of insulin plasma distribution, e.g., 0.1421 (liter/kg);

$m_b$: a weight (kg), e.g., 70kg;

Analytical solutions of plasma NPH concentration $I_N$ with respect to time $t$ can be obtained by solving the abovementioned equation set:

$$I_N =\exp(-(4^*t)/25)^*((1022699^*\exp((27^*t)/200))/163961000 +$$

$$(50681673^*\exp((793^*t)/5000))/4936028500 - (2182714053^*\exp((953^*t)/10000))/494945696200 -$$

$$(4449021^*\exp((1511^*t)/10000))/327887000 + 1682730209/1141906519000) \qquad (8)$$

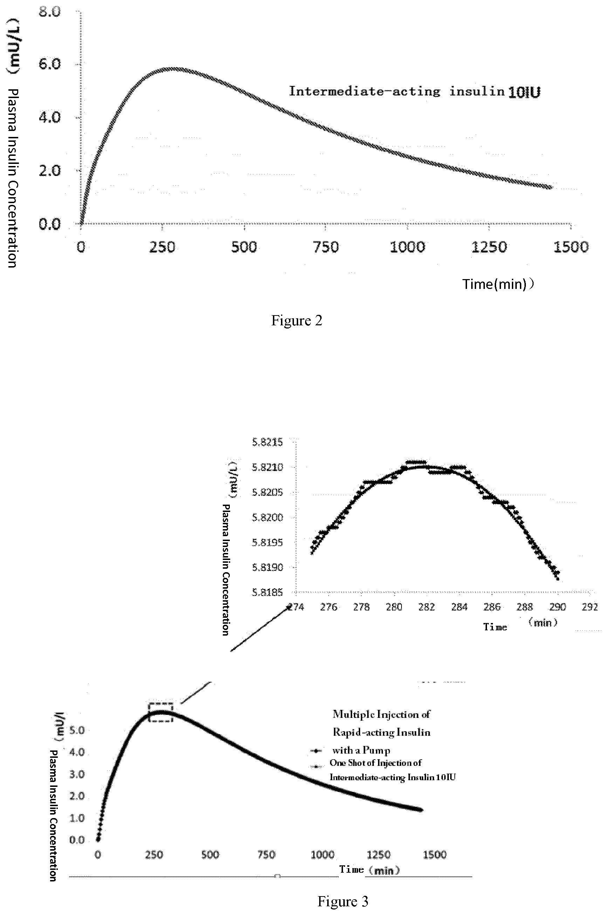

The insulin concentration in plasma at any moment after one shot of injection of the intermediate-acting insulin can be obtained according to the equation above. Figure 2 illustratively shows a curve of plasma concentration variation of a patient weighed 70kg when subcutaneously injected with 10IUNPH.

Next, the pharmacokinetic features of rapid-acting insulin are calculated.

The process of subcutaneous absorption of rapid-acting insulin is described using differential equations:

$$u_{total,mono}(t) =u_{mono}(t) \qquad (9)$$

$$\dot{x}_{dm}(t) = -(k_2+k_d)x_{dm}(t)+k_1x_h(t) +u_{m,NPH}(t) \qquad (5)$$

$$\dot{x}_i(t) = -(k_3+k_{d,i})x_i(t)+k_2x_{dm}(t) \qquad (6)$$

$$\dot{I}(t) = -nI(t)+k3\left\{\frac{x_i(t)}{V_im_b}\right\} \qquad (7)$$

Therein,

$U_{total,mono}(t)$ : a total rate (mU/min) of injecting rapid-acting insulin;
$u_{mono}(t)$: a rate (mU/min) of injecting monomeric/dimeric rapid-acting insulin;
With respect to other parameters, reference can be made to the previous text.

The following equations can be obtained by solving the abovementioned equation set:

$$x_{dmt} = x_{dm0} *\exp(-(53*t)/5000) \tag{10}$$

$$x_{it} =(20*\exp(-(321*t)/5000)*((613*x_{i0})/20 - (32489*x_{dm0})/5360 + (32489*x_{dm0}*\exp((67*t)/1250))/5360))/613 \tag{11}$$

$$I_{mt} =\exp(-(4*t)/25)*((32489*x_{dm0})/7156260 - (613*x_{i0})/9580 + I_{m0} - \exp((479*t)/5000)*((32489*x_{dm0})/2567440 - (613*x_{i0})/9580) + (32489*x_{dm0}*\exp((747*t)/5000))/4003920) \tag{12}$$

wherein,

$x_{dm0}$: an amount (mU) of subcutaneous monomeric/dimeric rapid-acting insulin at the moment of 0;
$x_{dmt}$: an amount (mU) of subcutaneous monomeric/dimeric rapid-acting insulin at the moment of t;
$x_{i0}$: an amount (mU) of interstitial rapid-acting insulin at the moment of 0;
$x_{it}$: an amount (mU) of interstitial rapid-acting insulin at the moment of t;
$I_{m0}$: a concentration (mU/L) of rapid-acting insulin in plasma at the moment of 0;
$I_{mt}$: a concentration (mU/L) of rapid-acting insulin in plasma at the moment of t;

The amount of subcutaneous and interstitial insulin and the insulin concentration in plasma at any moment after one shot of injection of the rapid-acting insulin can be respectively calculated through the abovementioned equations.

Next, an injection amount of an insulin pump at each time point is calculated.

The amount of injecting rapid-acting insulin with a pump can be calculated by a cyclic iterative method to analyze the equation set composed of equations (10) to (12) according to the concentration values of the intermediate-acting insulin NPH at each time point. Supposing that the pump injects once every three minutes, the specific calculation method is as follow:

(a) it is calculated that the plasma concentration of intermediate-acting insulin within three minutes is $I_{N3}$ = 0.07987mU/L according to equation (8);
(b) the amount of rapid-acting insulin in each location at the moment of 0 is obtained; supposing that the rapid-acting insulin $U_0$ is injected at the moment of 0, then the amount of the instantaneously subcutaneous rapid-acting insulin when insulin $U_0$ is injected at the moment of 0 is $x_{dm0}^+=U_0$; at this moment, the rapid-acting insulin has not yet arrived in the interstice and the plasma, so $x_{i0}$=0 in the interstice, and $I_{m0}$=0 in plasma;
(c) if it is desired that the rapid-acting insulin concentration in the 3$^{rd}$ minute reaches the intermediate-acting insulin level at this moment, then $I_{m3}=I_{N3}$=0.07987mU/L. If $x_{dm0}^+=U_0$, $x_{i0}$=0, $I_{m0}$=0, $I_{m3}$=0.07987mU/L are substituted into equation (12), and a value of 3min is assigned to t, a following equation can be obtained:

0.07987mU/L  =

$\exp(-(4*3)/25)*((32489*x_{dm0}^+)/7156260-(613*0)/9580+0-\exp((479*3)/5000)*((32489*x_{dm0}^+)/2567440-(613*0)/9580)+(32489*x_{dm0}^+*\exp((747*3)/5000))/4003920)$

In this equation, only $x_{dm0}^+$ is unknown, the equation is solved to obtain $x_{dm0}^+$=343.994mU, $U_0=x_{dm0}^+$=343.994mU, i.e., the injection amount of insulin at moment 0 is 343.994mU;
(d) the amount of rapid-acting insulin at each location in the 3$^{rd}$ minute is sought; it is recorded that the subcutaneous rapid-acting insulin before injection for the second time using the pump in the 3$^{rd}$ minute is in an amount of $x_{dm3}^-$, it can be obtained that $x_{dm3}^-$=333.227mU according to equation (10); it is recorded that the amount of interstitial rapid-acting insulin in the 3$^{rd}$ minute is $x_{i3}$, and it is obtained that $x_{i3}$=9.789mU according to equation (11); and it can be derived from step (c) that $I_{m3}$=0.079874mU/L;

(e) according to equation (8), it is calculated that the plasma concentration of the intermediate-acting insulin in the 6th minute is $I_{N6}$=0.2605mU/L;

(f) let the amount of injecting rapid-acting insulin in the 3rd minute be $U_3$, then $x_{dm3}{}^+=x_{dm3}{}^-+U_3$=333.227mU+$U_3$; it can be derived from step (d) that $x_{i3}$=9.789mU, $I_{m3}$=0.079874mU/L; it is calculated from step (e) that the plasma concentration of rapid-acting insulin after 3 minutes (i.e., in the 6th minute) should be $I_{m6}$=$I_{N6}$=0.2605mU/L. The plasma insulin concentration in the 6th minute is calculated on the basis of the amount of rapid-acting insulin at each location in the 3rd minute, and substituted into equation (12) to obtain the equation

$$0.2605\text{mU/L}=\exp(-(4*3)/25)*((32489*(333.227\text{mU}+U_3))/7156260-(613*9.789\text{mU})/9580+$$
$$0.079874\text{mU/L}-\exp((479*3)/5000)*((32489*(333.227\text{mU}+$$
$$U_3))/2567440-(613*9.789\text{mU})/9580)+(32489*(333.227\text{mU}+U_3)^+*\exp((747*3)/5000))/4003920)$$

in which only $U_3$ is unknown and the equation is solved to obtain $U_3$=20.2026mU, i.e., 20.2026mu should be injected in the 3rd minute, and $x_{dm3}{}^+=x_{dm3}{}^-+U_3$=353.4296mU after injection;

(g) steps (d) to (f) are repeated to calculate the injection amount of the insulin pump every three minutes. Reference can be made to Table 1, wherein the injection amount of the rapid-acting insulin of the insulin pump at each time point is displayed.

**Table 1　Injection Amount (mU) of Rapid-acting Insulin of an Insulin Pump at Each Time Point**

| Hours | Minutes | | | | | | | | | | | | | | | | | | | |
|---|---|---|---|---|---|---|---|---|---|---|---|---|---|---|---|---|---|---|---|---|
| | 0 | 3 | 6 | 9 | 12 | 15 | 18 | 21 | 24 | 27 | 30 | 33 | 36 | 39 | 42 | 45 | 48 | 51 | 53 | 57 |
| 0~1 | 344.66 | 16.96 | 12.90 | 18.84 | 16.66 | 20.70 | 19.64 | 22.45 | 22.05 | 24.05 | 24.02 | 25.47 | 25.63 | 26.71 | 26.97 | 27.79 | 28.08 | 28.72 | 29.01 | 29.50 |
| ~2 | 29.78 | 30.17 | 30.41 | 30.73 | 30.94 | 31.19 | 31.37 | 31.56 | 31.71 | 31.86 | 31.98 | 32.10 | 32.20 | 32.28 | 32.35 | 32.42 | 32.46 | 32.51 | 32.53 | 32.56 |
| ~3 | 32.57 | 32.58 | 32.57 | 32.57 | 32.55 | 32.53 | 32.50 | 32.47 | 32.44 | 32.40 | 32.35 | 32.30 | 32.25 | 32.20 | 32.14 | 32.08 | 32.01 | 31.94 | 31.87 | 31.80 |
| ~4 | 31.73 | 31.65 | 31.57 | 31.49 | 31.41 | 31.32 | 31.24 | 31.15 | 31.07 | 30.98 | 30.89 | 30.79 | 30.70 | 30.61 | 30.52 | 30.42 | 30.33 | 30.23 | 30.13 | 30.03 |
| ~5 | 29.94 | 29.84 | 29.74 | 29.64 | 29.54 | 29.44 | 29.34 | 29.24 | 29.14 | 29.04 | 28.94 | 28.83 | 28.73 | 28.63 | 28.53 | 28.43 | 28.32 | 28.22 | 28.12 | 28.02 |
| ~6 | 27.91 | 27.81 | 27.71 | 27.61 | 27.50 | 27.40 | 27.30 | 27.20 | 27.09 | 26.99 | 26.89 | 26.79 | 26.69 | 26.58 | 26.48 | 26.38 | 26.28 | 26.18 | 26.08 | 25.98 |
| ~7 | 25.88 | 25.78 | 25.68 | 25.58 | 25.48 | 25.38 | 25.28 | 25.18 | 25.08 | 24.98 | 24.88 | 24.79 | 24.69 | 24.59 | 24.49 | 24.40 | 24.30 | 24.20 | 24.11 | 24.01 |
| ~8 | 23.92 | 23.82 | 23.72 | 23.63 | 23.53 | 23.44 | 23.35 | 23.25 | 23.16 | 23.07 | 22.97 | 22.88 | 22.79 | 22.70 | 22.60 | 22.51 | 22.42 | 22.33 | 22.24 | 22.15 |
| ~9 | 22.06 | 21.97 | 21.88 | 21.79 | 21.70 | 21.61 | 21.53 | 21.44 | 21.35 | 21.26 | 21.18 | 21.09 | 21.00 | 20.92 | 20.83 | 20.75 | 20.66 | 20.58 | 20.49 | 20.41 |
| ~10 | 20.32 | 20.24 | 20.16 | 20.07 | 19.99 | 19.91 | 19.83 | 19.75 | 19.66 | 19.58 | 19.50 | 19.42 | 19.34 | 19.26 | 19.18 | 19.10 | 19.02 | 18.94 | 18.87 | 18.79 |
| ~11 | 18.71 | 18.63 | 18.56 | 18.48 | 18.40 | 18.33 | 18.25 | 18.17 | 18.10 | 18.02 | 17.95 | 17.87 | 17.80 | 17.73 | 17.65 | 17.58 | 17.51 | 17.43 | 17.36 | 17.29 |
| ~12 | 17.22 | 17.15 | 17.07 | 17.00 | 16.93 | 16.86 | 16.79 | 16.72 | 16.65 | 16.58 | 16.51 | 16.45 | 16.38 | 16.31 | 16.24 | 16.17 | 16.11 | 16.04 | 15.97 | 15.90 |
| ~13 | 15.84 | 15.77 | 15.71 | 15.64 | 15.58 | 15.51 | 15.45 | 15.38 | 15.32 | 15.25 | 15.19 | 15.13 | 15.06 | 15.00 | 14.94 | 14.88 | 14.81 | 14.75 | 14.69 | 14.63 |
| ~14 | 14.57 | 14.51 | 14.45 | 14.39 | 14.33 | 14.27 | 14.21 | 14.15 | 14.09 | 14.03 | 13.97 | 13.91 | 13.85 | 13.80 | 13.74 | 13.68 | 13.62 | 13.57 | 13.51 | 13.45 |
| ~15 | 13.40 | 13.34 | 13.28 | 13.23 | 13.17 | 13.12 | 13.06 | 13.01 | 12.95 | 12.90 | 12.85 | 12.79 | 12.74 | 12.69 | 12.63 | 12.58 | 12.53 | 12.47 | 12.42 | 12.37 |
| ~16 | 12.32 | 12.27 | 12.22 | 12.16 | 12.11 | 12.06 | 12.01 | 11.96 | 11.91 | 11.86 | 11.81 | 11.76 | 11.71 | 11.66 | 11.62 | 11.57 | 11.52 | 11.47 | 11.42 | 11.37 |
| ~17 | 11.33 | 11.28 | 11.23 | 11.19 | 11.14 | 11.09 | 11.05 | 11.00 | 10.95 | 10.91 | 10.86 | 10.82 | 10.77 | 10.73 | 10.68 | 10.64 | 10.59 | 10.55 | 10.50 | 10.46 |
| ~18 | 10.42 | 10.37 | 10.33 | 10.28 | 10.24 | 10.20 | 10.16 | 10.11 | 10.07 | 10.03 | 9.99 | 9.95 | 9.90 | 9.86 | 9.82 | 9.78 | 9.74 | 9.70 | 9.66 | 9.62 |
| ~19 | 9.58 | 9.54 | 9.50 | 9.46 | 9.42 | 9.38 | 9.34 | 9.30 | 9.26 | 9.22 | 9.18 | 9.14 | 9.11 | 9.07 | 9.03 | 8.99 | 8.95 | 8.92 | 8.88 | 8.84 |
| ~20 | 8.80 | 8.77 | 8.73 | 8.69 | 8.66 | 8.62 | 8.59 | 8.55 | 8.51 | 8.48 | 8.44 | 8.41 | 8.37 | 8.34 | 8.30 | 8.27 | 8.23 | 8.20 | 8.16 | 8.13 |
| ~21 | 8.10 | 8.06 | 8.03 | 7.99 | 7.96 | 7.93 | 7.89 | 7.86 | 7.83 | 7.80 | 7.76 | 7.73 | 7.70 | 7.67 | 7.63 | 7.60 | 7.57 | 7.54 | 7.51 | 7.47 |
| ~22 | 7.44 | 7.41 | 7.38 | 7.35 | 7.32 | 7.29 | 7.26 | 7.23 | 7.20 | 7.17 | 7.14 | 7.11 | 7.08 | 7.05 | 7.02 | 6.99 | 6.96 | 6.93 | 6.90 | 6.87 |
| ~23 | 6.84 | 6.82 | 6.79 | 6.76 | 6.73 | 6.70 | 6.67 | 6.65 | 6.62 | 6.59 | 6.56 | 6.53 | 6.51 | 6.48 | 6.45 | 6.43 | 6.40 | 6.37 | 6.35 | 6.32 |
| ~24 | 6.29 | 6.27 | 6.24 | 6.21 | 6.19 | 6.16 | 6.14 | 6.11 | 6.08 | 6.06 | 6.03 | 6.01 | 5.98 | 5.96 | 5.93 | 5.91 | 5.88 | 5.86 | 5.83 | 5.81 |

In order to verify the coincidence of plasma concentrations of one shot of injecting both rapid-acting insulin and intermediate-acting insulin using the pump, the plasma concentrations of one shot of injecting both the rapid-acting insulin and the intermediate-acting insulin using the pump are respectively calculated every six seconds. Results show that the difference value therebetween is less than 1% after one minute, and is less than 0.1% after three minutes. Figure 3 shows both plasma concentration curves, and indicates relatively high coincidence.

According to the examples above, the injection amounts of the pump at respective time points can be calculated so as to realize transformation of a scheme of injecting intermediate-acting or long-acting insulin into a scheme of injecting rapid-acting insulin. In other embodiment modes, it can be realized that an injection scheme of intermediate-acting or long-acting insulin is transformed into an injection scheme of short-acting insulin, or that transformation of other injection schemes can be realized. Table 2 shows kinetic parameters of various insulin that are used for simulation. Table 2 shows kinetic parameters of various types of insulin products, wherein the values thereof are medians and can be used for reference.

**Table 2 Table of Kinetic Parameters of Various Types of Insulin**

| Type of Insulin | $\alpha_{crys}$ | $\alpha_{hex}$ | $\alpha_{dm}$ | $k_{crys}$ | $K_1$ | $K_2$ | $K_3$ | Source |
|---|---|---|---|---|---|---|---|---|
| Rapid-Acting MI | 0 | 0 | 1.000 | - | - | 0.0106 | 0.0613 | 5 |
| Short-ActingRI | 0 | 0.300 | 0.700 | - | 0.0250 | 0.0089 | 0.0618 | 6 |
| Intermediate-Acting NPH | 0.9432 | 0.017 | 0.040 | 0.0014 | 0.0250 | 0.0089 | 0.0618 | 7 |
| Intermediate-Acting Lente | 0.9447 | 0.017 | 0.039 | 0.0037 | 0250 | 0.0089 | 0.0618 | 8 |
| Long-Acting Ultralente | 1.0000 | 0 | 0 | 0.0013 | 0.0018 | 0.0089 | 0.0618 | 9 |
| Long-Acting Glargine | 0.9462 | 0.016 | 0.038 | 0.0008 | 0.0084 | 0.0089 | 0.0618 | 10 |

In the abovementioned examples, the amounts of rapid-acting insulin in various locations can also be obtained without equations, but measured through proper detection methods (e.g., ACS 180PLUS); the injection interval can also be set as other suitable values, e.g., two minutes, five minutes or ten minutes, rather than 3 minutes.

In a further example, the displacement distance of a pump can be further monitored in real time using, for example, a displacement transducer, thereby monitoring an injection amount to achieve the purpose of accurate injection.

In some other embodiment modes of the present invention, the injection schemes of other preparations can also be transformed to obtain injections schemes that are more beneficial for use in the injection pumps, thereby providing great convenience to patients.

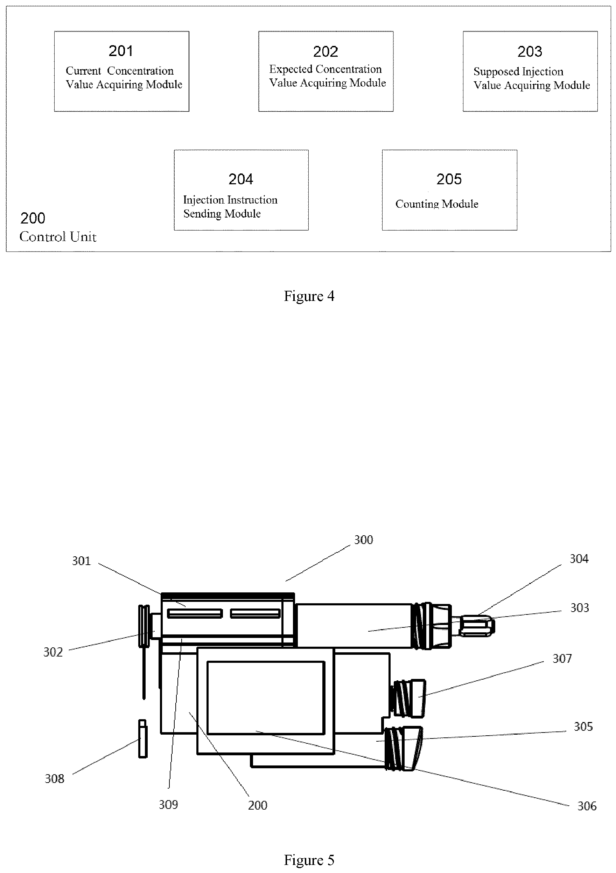

Figure 4 shows a functional diagram of a control unit for an injection pump according to an embodiment mode of the present invention. As illustrated in the figure, a control unit 200 comprises a current concentration value acquiring module 201, an expected concentration value acquiring module 202, a supposed injection value acquiring module 203, an injection instruction sending module 204 and a time counting module 205. An example of using insulin as a preparation is made again. The current concentration value module 201 is used to acquire a current subcutaneous insulin value and a current interstitial insulin value at a current moment of an individual to be injected with a preparation. The expected concentration value acquiring module 202 acquires an expected plasma insulin concentration value at a predetermined next moment based on a first insulin injection scheme. The supposed injection value acquiring module 203 acquires a supposed injection value at a current moment based on a second insulin injection scheme, an expected plasma insulin concentration value at a next moment, a current subcutaneous insulin value and a current interstitial insulin value. The injection instruction sending module 204 sends out an instruction to inject a preparation into an individual using the pump according the supposed injection value. The time counting module 205 counts the time, and sends out a signal to stop injection when the time from the first injection reaches a lasting time length of the first injection scheme.

The time counting module 205 can be realized through, e.g., a system clock of the control unit. It can also be realized through, for example, a counter; then in such circumstance, when the number of times of predetermined injections is reached, the counter sends out a signal to stop injection. In some embodiment modes, let the time interval between each injection be unchanged, then the number of times of predetermined injections can be obtained using a division method simply based on the duration of the first injection scheme.

It should be understood that Figure 4 only divides each module of control unit 200 functionally rather than dividing the control unit physically. In fact, the functions of some modules might be integrated into a chip, or even the entire control unit is only embodied as a chip or integrated as a part in a chip.

Figure 5 illustratively presents an injection pump according to an embodiment mode of the present invention. As illustrated in the figure, the pump body of an injection pump 300 comprises a rod valve push 301, a motor assembly 302, an injection cavity 303 and an injection head 304. The motor assembly 302 is arranged at the rear end of the rod valve push 301 to provide power to drive the rod valve push 301. The injection cavity 303 is arranged at the front end of the rod valve push 301, wherein a preparation is stored, and when the rod valve push 301 is subjected to a pressure, the preparation is injected outwardly from an injection head 304 at the front end of the injection cavity 303. A displacement transducer 309 can be arranged below the rod valve push 301 to monitor the displacement distance of a pump. An injection pump 300 further comprises a battery cavity 305 below the pump body and some other electronic components, e.g., a liquid crystal panel 306 arranged in the middle for information interaction with a user, and e.g., a USB interface 307 arranged at one side of the liquid crystal panel 306 as a communication interface. The injection pump 300 further comprises an alarm component 308 that is detachably connected to send out alarms in special conditions. A control unit 200 in the form of a control panel is arranged inside the injection pump 300. The electronic components mentioned above can be arranged as members separated from the control unit 200 in space, or can be integrated into a control panel as a part of the control unit 200.

**Example 1:**

The present invention is applied to an SD rat used for experiment. The injection accuracy of the injection pump in an animal experiment is studied in the present invention, feasibility of the present invention is further discussed by comparing the pharmacokinetic curve between the injection accuracy and subcutaneous injection of insulin.

Method: SD rats are used and STZ is used for once large dose injection into an abdominal cavity. After one week, a blood glucose level is detected. If it is greater than 11.6mmol/L, then it is prompted that a T1DM rat model is successfully established. The T1DM rats are randomly divided into an NPH single subcutaneous injection group, an RI injection pump continuous subcutaneous injection group, and a blank control group. During the 4-hour experiment, sampling is conducted at each blood collection point and insulin concentration thereof is detected, respectively. The injection amount of the injection pump is also measured every 15 minutes according to the scheme and compared with the theoretical expected value.

I. Animals in Experiments

1.1 The strain comes from T1DM diabetic SD rats purchased from Nanjing BetterBiotechnology Co., Ltd.

1.2 Gender and Week Age: male, 7-8 weeks.

1.3 The feeding environment is at the temperature between 20 and 25 °C, in a humidity between 40% and 70% or so. The rats eat and drink freely, and the rat cage is cleaned regularly.

2. Injection Pumps to be Tested

There are totally 8 injection pumps used in the experiment, which are encoded with P001 to P008 and produced by Shanghai Zensun Science and Technology Development Co., Ltd.

3. Experimental Materials

Novolin R Cartridge 100IU/ml, Novo Nordisk (China) Pharmaceutical Co., Ltd; Novolin N Cartridge 100IU/ml, Novo Nordisk (China) Pharmaceutical Co., Ltd.

4. Experimental Method

4.1 Preparation of a T1DM Rat Model

After ambrosia for 24 hours of SD rats that weigh 200g, the dosage of 70mg/kg is respectively administered to each group through once abdominal injection.

Model Forming Standard: the rat tail venous blood glucose value is measured using a glucometer after 72 hours from administration, a random glucose value equal to or greater than 11.6mmol/L serves as a model forming standard for diabetic rats. The random glucose value that is actually measured after molding is equal to or greater than 20mmol/L.

4.2 Grouping and Administration

After the model is successfully established, T1DM rats are randomly divided into NPH single subcutaneous injection group, an injection pump using group, and a blank control group. As for the NPH single subcutaneous injection group, Novolin N is used for single subcutaneous injection, and administered in a dose of 5IU/kg; in the RI injection pump continuously subcutaneous injection group, an NPH sample of Novolin R in the kinetic progress is administered for subcutaneously continuous pumping; the black control group is administered and injected with normal saline using the pump for subcutaneously continuous pumping.

4.3 Measurement of Insulin

Blood samples are collected and the insulin amounts are detected 30 minutes, 45 minutes, 60 minutes, 75 minutes, 90 minutes, 105 minutes, 120 minutes, 135 minutes, 150 minutes, 180 minutes and 240 minutes after treatment by administration. A Roche electrochemical luminescence instrument cobas e601 is used for insulin detection, which is produced by a Roche company of diagnosis products and gauged by Guangzhou Kingmed Medical Detection Center.

5. Experimental Results

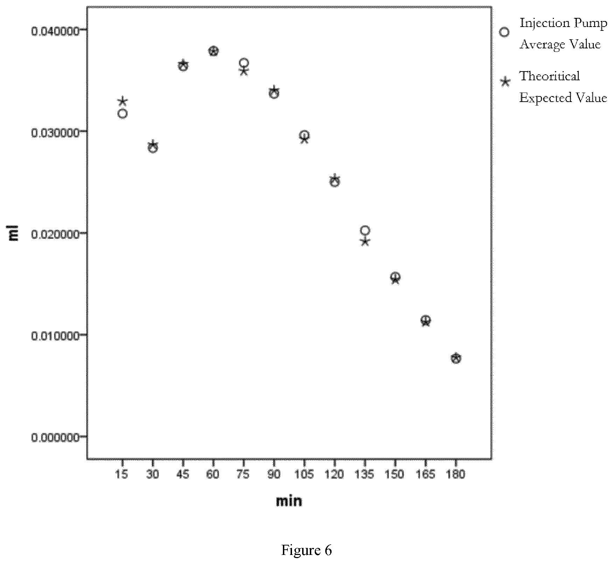

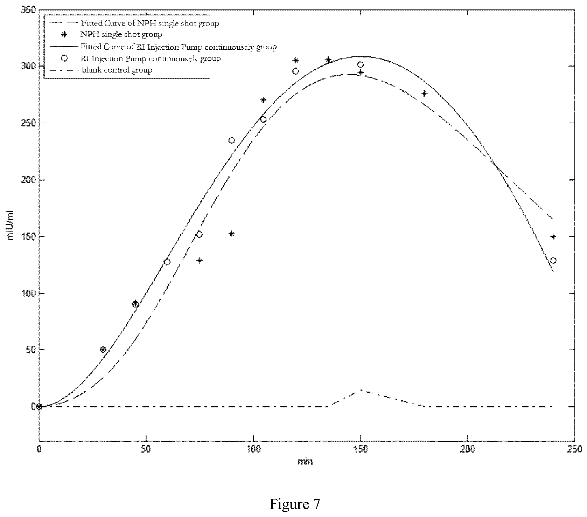

The injection amounts of the injection pump every 15 minutes in the experiment are substantially consistent with theoretical expectations, see Figure 6. As compared with NPH single subcutaneous injection, the continuously subcutaneous injection group using an RI injection pump shows the reliability in the process of controlling *in vivo* metabolic process of insulin from *in vitro* administration, the peak reaching time and peak reaching concentration of both conditions being substantially consistent; the blank control group proves that the mechanical results of the injection pump would not produce any impact on insulin variation, see Figure 7.

6. Conclusion

After using the injection pump of the present patent, the curves of insulin *in vivo* concentration can be basically controlled as time changes.
The present invention is set forth above through specific embodiment modes. It should be understood that the abovementioned descriptions are descriptive rather than restrictive. For example, the features of the abovementioned contents can be used solely, or can be used in any combination as long as they do not go beyond the scope of the present invention. Moreover, without departing from the spirit of the present invention, amendments can be made to the embodiment modes to adapt to specific circumstances. Although the specific elements and process in the text define various embodiment modes, they are not restrictive but demonstrative. By reading the abovementioned description, many other examples would be obvious to persons skilled in the art. Thus, the scope of the present invention should be determined by considering the claims as well as the entirely equivalent scope covered by such kind of claims.

**Claims**

**1.** A method of controlling an insulin injection pump, comprising:

(1) obtaining a first expected plasma insulin concentration value of an individual to be injected with insulin at a predetermined first moment based on a first insulin injection scheme;
(2) calculating an initial supposed injection value at an initial moment based on a second insulin injection scheme and the first expected plasma insulin concentration value, wherein a time interval between the initial moment and the first moment is defined as an injection time interval;
(3) injecting insulin into the individual using the pump according to the initial supposed injection value;
(4) obtaining a first moment subcutaneous monomeric/dimeric insulin value and a first moment interstitial insulin value at the first moment based on a second insulin injection scheme;
(5) obtaining a second expected plasma insulin concentration value at a predetermined second moment based on the first insulin injection scheme, wherein an interval between the second moment and the first moment is equal to the injection time interval;
(6) calculating a first supposed injection value of the first moment based on the second insulin injection scheme, the second expected plasma insulin concentration value, the first moment subcutaneous monomeric/dimeric insulin value and the first moment interstitial insulin value;
(7) injecting insulin into the individual using the pump according to the first supposed injection value; and
(8) obtaining a supposed injection value at each moment by iteration based on steps (4) to (7) and injecting insulin into the individual using the pump according to the supposed injection value until an N-th supposed injection value at a N-th moment is reached, wherein a product of N multiplied by the injection time interval is equal to a lasting time length of the first insulin injection scheme.

**2.** A control unit for controlling an insulin injection pump, comprising:

an expected plasma insulin concentration value acquiring module, which is configured to obtain a first expected plasma insulin concentration value for an individual to be injected with insulin at a predetermined first moment based on a first insulin injection scheme, and a second expected plasma insulin concentration value at a predetermined second moment, until an N-th expected plasma insulin concentration value at a predetermined N-th moment is reached, wherein a time interval between an initial moment and the first moment is defined as an injection time interval, a time interval between the second moment and the first moment is equal to the injection time interval, each time interval between each moment and a next moment is equal to the injection time interval, while a product of N multiplied by the injection time interval is equal to a lasting time length of the first insulin injection scheme;

a current subcutaneous and interstitial insulin concentration value acquiring module, which is configured to obtain a current subcutaneous insulin value and a current interstitial insulin value at a current moment;

a supposed injection value acquiring module, which is configured to obtain a supposed injection value at a current moment based on a second insulin injection scheme, an expected plasma insulin concentration value at a next moment, the current subcutaneous insulin value and the current interstitial insulin value;

an injection instruction sending module, which is configured to send instructions to inject insulin into the individual using the pump according to the supposed injection value; and

a counting module, which is configured to send out a signal to stop injection when an N-th injection is accomplished.

3. The control unit according to claim 2, **characterized in that** the control unit further comprises a displacement transducer for monitoring a displacement distance of a pump in real time.

4. An injection pump for injecting insulin, comprising a pump body and the control unit according to any of claims 2 and 3, wherein the control unit is arranged in the pump body.

5. A method of controlling an insulin injection pump, comprising:

(1) obtaining a first expected plasma insulin concentration value of an individual to be injected with insulin at a predetermined first moment based on a first insulin injection scheme;
(2) calculating an initial supposed injection value at an initial moment based on a second insulin injection scheme and the first expected plasma insulin concentration value;
(3) injecting insulin into the individual using the pump according to the initial supposed injection value;
(4) obtaining a first moment subcutaneous monomeric/dimeric insulin value and a first moment interstitial insulin value at the first moment based on a second insulin injection scheme;
(5) obtaining a second expected plasma insulin concentration value at a predetermined second moment based on the first insulin injection scheme;
(6) calculating a first supposed injection value of the first moment based on the second insulin injection scheme, the second expected plasma insulin concentration value, the first moment subcutaneous monomeric/dimeric insulin value and the first moment interstitial insulin value;
(7) injecting insulin into the individual using the pump according to the first supposed injection value;
(8) obtaining a supposed injection value at each moment by iteration based on steps (4) to (7) and injecting insulin into the individual using the pump according to the supposed injection value until the time experienced from a first injection reaches a lasting time length of a first insulin injection scheme.

6. The method according to claim 5, **characterized in that** an interval between the first moment and the initial moment is equal to an interval between the second moment and the first moment.

7. The method according to claim 5, **characterized in that** the time intervals between every two injections are fixed.

8. A control unit for controlling an insulin injection pump, comprising:

an expected plasma insulin concentration value acquiring module, which is configured to obtain a first expected plasma insulin concentration value for an individual to be injected with insulin at a predetermined first moment based on a first insulin injection scheme, and a second expected plasma insulin concentration value at a predetermined second moment, until an N-th expected plasma insulin concentration value at a predetermined N-th moment is reached, wherein N refers to times of predetermined injections;

a current subcutaneous and interstitial insulin concentration value acquiring module, which is configured to obtain a current subcutaneous insulin value and a current interstitial insulin value at a current moment;

a supposed injection value acquiring module, which is configured to obtain a supposed injection value at a current moment based on a second insulin injection scheme, an expected plasma insulin concentration value at a next moment, the current subcutaneous insulin value and the current interstitial insulin value;
an injection instruction sending module, which is configured to send instructions to inject insulin into the individual using the pump according to the supposed injection value; and
a time counting module, which is configured to send out a signal to stop injection when the time experienced from a first injection reaches a lasting time length of a first injection scheme.

9. The control unit according to claim 8, **characterized in that** the time counting module is achieved by a counter, wherein when a number of times for predetermined injection is reached, the time counting module sends out a signal to stop injection.

10. An injection pump for injecting insulin, comprising a pump body and the control unit according to any of claims 8 to 9, wherein the control unit is arranged inside the pump body.

```
┌─────────────────────────────────────────┐
│                  101                      │ ◄─────────────┐
│                                           │               │
│    Obtaining a current concentration value│               │
└─────────────────────────────────────────┘               │
                    │                                       │
                    ▼                                       │
┌─────────────────────────────────────────┐               │
│                  102                      │               │
│                                           │               │
│   Obtaining an expected concentration value│              │
│   at a next moment based on a first injection│            │
└─────────────────────────────────────────┘               │
                    │                                       │
                    ▼                                       │
┌─────────────────────────────────────────┐               │
│                  103                      │               │
│                                           │               │
│    Obtaining a supposed injection value at a│             │
│    current moment based on a second        │             │
└─────────────────────────────────────────┘               │
                    │                                       │
                    ▼                                       │
┌─────────────────────────────────────────┐               │
│                  104                      │               │
│                                           │               │
│   Injecting according to a supposed injection│            │
└─────────────────────────────────────────┘               │
                    │                                       │
                    ▼                                       │
              ╱─────────────╲                               │
           ╱       105         ╲                      N     │
        ╱  Whether a lasting time length of the ╲──────────┘
        ╲  first injection scheme is achieved?  ╱
           ╲                 ╱
              ╲─────────────╱
                    │
                    │ Y
                    ▼
          ╭─────────────────────╮
          │                      │
          │         END          │
          │                      │
          ╰─────────────────────╯
```

Figure 1

Figure 2

Figure 3

| 201 Current Concentration Value Acquiring Module | 202 Expected Concentration Value Acquiring Module | 203 Supposed Injection Value Acquiring Module |
| 204 Injection Instruction Sending Module | 205 Counting Module | |

200
Control Unit

Figure 4

300

301

304

303

302

307

305

308

309    200

306

Figure 5

Figure 6

Figure 7

# INTERNATIONAL SEARCH REPORT

| | International application No. |
|---|---|
| | PCT/CN2015/094538 |

**A. CLASSIFICATION OF SUBJECT MATTER**

A61M 5/142 (2006.01) i

According to International Patent Classification (IPC) or to both national classification and IPC

**B. FIELDS SEARCHED**

Minimum documentation searched (classification system followed by classification symbols)

A61M 5/-; A61B 5/-; G06F

Documentation searched other than minimum documentation to the extent that such documents are included in the fields searched

Electronic data base consulted during the international search (name of data base and, where practicable, search terms used)

CNPAT, WPI, EPODOC: insulin, trypsin, blood sugar, plasma, pump, simulat+, control+, concentrat+, expectation, long acting, short acting, medium acting, inject+, dynamics

**C. DOCUMENTS CONSIDERED TO BE RELEVANT**

| Category* | Citation of document, with indication, where appropriate, of the relevant passages | Relevant to claim No. |
|---|---|---|
| A | CN 101866387 A (SHANGHAI YANGPU CENTRAL HOSPITAL) 20 October 2010 (20.10.2010) description, paragraphs [0015]-[0050] | 1-10 |
| A | WO 2013032965 A1 (UNIVERSITY OF VIRGINIA PATENT FOUNDATION) 07 March 2013 (07.03.2013) the whole document | 1-10 |
| A | WO 2010091102 A1 (ABBOTT DIABETES CARE INC.) 12 August 2010 (2010-08-12) the whole document | 1-10 |
| A | WO 2014008574 A1 (THOMSON, CAREN FRANCES) 16 January 2014 (16.01.2014) the whole document | 1-10 |

☐ Further documents are listed in the continuation of Box C.  ☒ See patent family annex.

| | |
|---|---|
| * Special categories of cited documents:<br><br>"A" document defining the general state of the art which is not considered to be of particular relevance<br><br>"E" earlier application or patent but published on or after the international filing date<br><br>"L" document which may throw doubts on priority claim(s) or which is cited to establish the publication date of another citation or other special reason (as specified)<br><br>"O" document referring to an oral disclosure, use, exhibition or other means<br><br>"P" document published prior to the international filing date but later than the priority date claimed | "T" later document published after the international filing date or priority date and not in conflict with the application but cited to understand the principle or theory underlying the invention<br><br>"X" document of particular relevance; the claimed invention cannot be considered novel or cannot be considered to involve an inventive step when the document is taken alone<br><br>"Y" document of particular relevance; the claimed invention cannot be considered to involve an inventive step when the document is combined with one or more other such documents, such combination being obvious to a person skilled in the art<br><br>"&"document member of the same patent family |

| Date of the actual completion of the international search | Date of mailing of the international search report |
|---|---|
| 26 January 2016 | 04 February 2016 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>LI, Xinchen<br><br>Telephone No. (86-10) 82245483 |

Form PCT/ISA /210 (second sheet) (July 2009)

**INTERNATIONAL SEARCH REPORT**
Information on patent family members

| International application No. |
| --- |
| PCT/CN2015/094538 |

| Patent Documents referred in the Report | Publication Date | Patent Family | Publication Date |
| --- | --- | --- | --- |
| CN 101866387 A | 20 October 2010 | CN 101866387 B | 26 September 2012 |
| WO 2013032965 A1 | 07 March 2013 | JP 2014534483 A | 18 December 2014 |
| | | AU 2012300331 A1 | 17 April 2014 |
| | | US 2015190098 A1 | 09 July 2015 |
| | | CA 2846854 A1 | 07 March 2013 |
| | | EP 2748747 A4 | 08 July 2015 |
| | | RU 2014111290 A | 27 October 2015 |
| | | CN 103907116 A | 02 July 2014 |
| | | EP 2748747 A1 | 02 July 2014 |
| WO 2010091102 A1 | 12 August 2010 | EP 2394217 A1 | 14 December 2011 |
| | | CN 102308278 A | 04 January 2012 |
| | | EP 2393419 A4 | 15 October 2014 |
| | | US 2010198142 A1 | 05 August 2010 |
| | | CN 102300501 A | 28 December 2011 |
| | | WO 2010091129 A1 | 12 August 2010 |
| | | CN 102300501 B | 08 April 2015 |
| | | EP 2393419 A1 | 14 December 2011 |
| WO 2014008574 A1 | 16 January 2014 | CA 2876250 A1 | 16 January 2014 |
| | | US 2015142325 A1 | 21 May 2015 |
| | | CN 104620244 A | 13 May 2015 |
| | | HK 1205576 A1 | 18 December 2015 |
| | | US 2014128834 A1 | 08 May 2014 |
| | | EP 2873014 A1 | 20 May 2015 |

Form PCT/ISA /210 (patent family annex) (July 2009)